# EUROPEAN PATENT APPLICATION

(11) **EP 4 767 941 A1**
(43) Date of publication of application: **01.07.2026**
(21) Application number: 25793322.6
(22) Date of filing: 24.03.2025
(51) Int. Cl.: A61B 5/318, B62D 1/04, A61B 5/346, A61B 5/00

(54) **ELECTROCARDIOGRAPHIC DATA MONITORING METHOD AND DEVICE, VEHICLE, AND STORAGE MEDIUM**

(30) Priority: 25.04.2024 CN 202410509836
(71) Applicant: Guangzhou Automobile Group Co., Ltd., Guangzhou, Guangdong 510030 (CN)
(72) Inventor: ZHUANG, Zhenming, Guangzhou, Guangdong 510030 (CN); LI, Yujin, Guangzhou, Guangdong 510030 (CN); LIU, Xitao, Guangzhou, Guangdong 510030 (CN); ZHAN, Denghui, Guangzhou, Guangdong 510030 (CN); WU, Huiyan, Guangzhou, Guangdong 510030 (CN); HUANG, Shen, Guangzhou, Guangdong 510030 (CN)
(74) Representative: Lucidity IP Ltd
(86) International application number: PCT/CN2025/084430
(87) International publication number: WO 2025/223124

(57) **Abstract**

The present application discloses an electrocardiogram (ECG) data monitoring method, an apparatus, a vehicle, and a storage medium. The method includes: during a dynamic driving process of a vehicle, in response to a data acquisition instruction, acquiring a steering wheel angle signal of the vehicle and acquiring a target image by an image acquisition device, wherein the target image being an image of a driver's hands holding the steering wheel, and a plurality of ECG monitoring sensors are arranged at intervals on the steering wheel; based on the steering wheel angle signal and the target image, determining a first sensor group and a second sensor group from the plurality of ECG monitoring sensors; acquiring first ECG data of the driver by the first sensor group, and acquiring second ECG data of the driver by the second sensor group; and based on the first ECG data, the second ECG data, and preset ECG data, determining target ECG data of the driver. Through the above method, the reliability of the target ECG data of the driver may be improved.

## Description

### CROSS-REFERENCE TO RELATED APPLICATION

The present application claims the priority of the Chinese patent application No. 2024105098368, filed on April 25, 2024, the entire contents of which are incorporated herein by reference for all purposes.

### TECHNICAL FIELD

The present application belongs to the technical field of vehicles, and in particular, relates to an electrocardiogram data monitoring method, an apparatus, a vehicle, and a storage medium.

### BACKGROUND

With the continuous growth in the number of vehicles in China, road traffic safety issues are becoming increasingly severe. Data indicates that the majority of traffic accidents are caused by the drivers themselves. In particular, accidents resulting in vehicle damage and fatalities occasionally occur due to sudden onset of cardiovascular disease in drivers. Therefore, it is necessary to monitor the vital signs of the driver in order to promptly understand the driver's driving state and physical condition, thereby effectively improving the driver's driving safety. Among these, monitoring of the driver's electrocardiogram (ECG) signal is extremely important. In related ECG monitoring methods, due to the movement of the driver's hands during dynamic driving of the vehicle, the reliability of the collected ECG data still requires improvement.

### SUMMARY

In view of the above problems, the present application proposes an electrocardiogram (ECG) data monitoring method, an apparatus, a vehicle, and a storage medium to address the above problems.

In a first aspect, an embodiment of the present application provides an electrocardiogram (ECG) data monitoring method including: during a dynamic driving process of a vehicle, in response to a data acquisition instruction, acquiring a steering wheel angle signal of the vehicle and acquiring a target image by an image acquisition device, wherein the target image being an image of a driver's hands holding the steering wheel, and a plurality of ECG monitoring sensors are arranged at intervals on the steering wheel; based on the steering wheel angle signal and the target image, determining a first sensor group and a second sensor group from the plurality of ECG monitoring sensors, wherein the first sensor group includes at least two ECG monitoring sensors each having a coverage greater than a coverage threshold, and the second sensor group includes at least two ECG monitoring sensors each having a coverage less than the coverage threshold; acquiring first ECG data of the driver by the first sensor group, and acquiring second ECG data of the driver by the second sensor group; and based on the first ECG data, the second ECG data, and preset ECG data, determining target ECG data of the driver, wherein the preset ECG data is obtained by analyzing basic ECG data of the driver collected by an ECG acquisition module when the vehicle is in a stationary state.

In a second aspect, an embodiment of the present application provides an electrocardiogram (ECG) data monitoring apparatus including: a first data acquisition unit configured to: during a dynamic driving process of a vehicle, in response to a data acquisition instruction, acquire a steering wheel angle signal of the vehicle and acquire a target image by an image acquisition device with the target image being an image of a driver's hands holding the steering wheel, wherein a plurality of ECG monitoring sensors are arranged at intervals on the steering wheel; a first determination unit configured to, based on the steering wheel angle signal and the target image, determine a first sensor group and a second sensor group from the plurality of ECG monitoring sensors, wherein the first sensor group includes at least two ECG monitoring sensors each having a coverage greater than a coverage threshold, and the second sensor group includes at least two ECG monitoring sensors each having a coverage less than the coverage threshold; a second data acquisition unit configured to acquire first ECG data of the driver by the first sensor group and acquire second ECG data of the driver by the second sensor group; and a second determination unit configured to determine target ECG data of the driver based on the first ECG data, the second ECG data, and preset ECG data, wherein the preset ECG data is obtained by analyzing basic ECG data of the driver collected by an ECG acquisition module when the vehicle is in a stationary state.

In a third aspect, an embodiment of the present application provides a vehicle including: one or more processors and a memory; and one or more programs, wherein the one or more programs are stored in a memory and configured to be executed by the one or more processors, the one or more programs being configured to perform the methods described above.

In a fourth aspect, an embodiment of the present application provides a computer-readable storage medium storing program codes, wherein when executed, the program codes perform the methods described above.

Embodiments of the present application provide an electrocardiogram (ECG) data monitoring method, an apparatus, a vehicle, and a storage medium. During a dynamic driving process of a vehicle, in response to a data acquisition instruction, a steering wheel angle signal is acquired, and a target image is acquired by an image acquisition device. The target image is an image of the driver's hands holding the steering wheel. A plurality of ECG monitoring sensors are arranged at intervals on the steering wheel. Then, based on the steering wheel angle signal and the target image, a first sensor group and a second sensor group are determined from the plurality of ECG monitoring sensors, with the first sensor group including at least two ECG monitoring sensors each having a coverage greater than a coverage threshold, and the second sensor group including at least two ECG monitoring sensors each having a coverage less than the coverage threshold. Next, first ECG data of the driver is acquired by the first sensor group and second ECG data of the driver is acquired by the second sensor group. Finally, the target ECG data of the driver is determined based on the first ECG data, the second ECG data, and preset ECG data. The preset ECG data is obtained by analyzing basic ECG data of the driver collected by an ECG acquisition module when the vehicle is in a stationary state. Through the above method, the accuracy and stability of monitoring ECG data of the driver can be improved, thereby enhancing the reliability of the target ECG data of the driver.

### BRIEF DESCRIPTION OF THE DRAWINGS

To more clearly illustrate the technical solutions in the embodiments of the present application, the accompanying drawings required for describing the embodiments will be briefly introduced below. Apparently, the accompanying drawings in the following description are only some embodiments of the present application, and for those of ordinary skill in the art other drawings can be obtained based on these accompanying drawings without creative effort.
FIG. 1 shows a flowchart of an ECG data monitoring method in an embodiment of the present application;
FIG. 2 shows a flowchart of an ECG data monitoring method in another embodiment of the present application;
FIG. 3 shows a flowchart of an ECG data monitoring method in yet another embodiment of the present application;
FIG. 4 shows a structural block diagram of an ECG data monitoring apparatus in an embodiment of the present application;
FIG. 5 shows a structural block diagram of a vehicle in an embodiment of the present application for executing an ECG data monitoring method in an embodiment of the present application; and
FIG. 6 shows a storage unit in an embodiment of the present application, which is used for saving or carrying a program code for implementing an ECG data monitoring method in an embodiment of the present application.

### DETAILED DESCRIPTION

The technical solutions in the embodiments of the present application will be clearly and completely described below in conjunction with the accompanying drawings in the embodiments of the present application. Obviously, the described embodiments are only some, not all, embodiments of the present application. All other embodiments obtained by those of ordinary skill in the art based on the embodiments of the present application without creative efforts shall fall within the protection scope of the present application.

An embodiment of the present application proposes an electrocardiogram (ECG) data monitoring method, an apparatus, a vehicle, and a storage medium. During a dynamic driving process of a vehicle, in response to a data acquisition instruction, a steering wheel angle signal of the vehicle is acquired, and a target image is acquired by an image acquisition device. The target image is an image of the driver's hands holding the steering wheel. A plurality of ECG monitoring sensors are arranged at intervals on the steering wheel. Then, based on the steering wheel angle signal and the target image, a first sensor group and a second sensor group are determined from the plurality of ECG monitoring sensors, with the first sensor group including at least two ECG monitoring sensors each having a coverage greater than a coverage threshold, and the second sensor group including at least two ECG monitoring sensors each having a coverage less than the coverage threshold. Next, first ECG data of the driver is acquired by the first sensor group, and second ECG data of the driver is acquired by the second sensor group. Finally, target ECG data of the driver is determined based on the first ECG data, the second ECG data, and preset ECG data. Here, the preset ECG data is obtained by analyzing basic ECG data of the driver collected by an ECG acquisition module, when the vehicle is in a stationary state. Through the above method, accuracy and stability of monitoring ECG data of the driver can be improved, thereby enhancing the reliability of the target ECG data of the driver.

Some embodiments of the present application will be described in detail below with reference to the accompanying drawings.

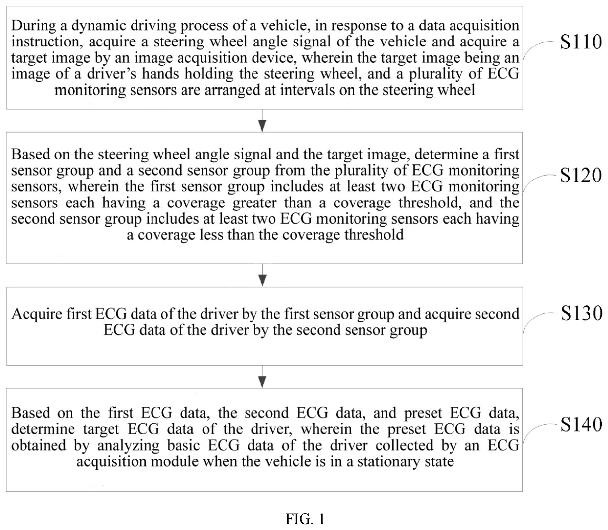

Referring to FIG. 1, an embodiment of the present application provides an electrocardiogram (ECG) data monitoring method including the following steps.

In step S110, during a dynamic driving process of a vehicle, in response to a data acquisition instruction, a steering wheel angle signal of the vehicle is acquired, and a target image is acquired by an image acquisition device. The target image is an image of the driver's hands holding the steering wheel. A plurality of ECG monitoring sensors are arranged at intervals on the steering wheel.

In an embodiment of the present application, the data acquisition instruction is an instruction for starting ECG data acquisition. The data acquisition instruction may be an instruction triggered when a specified operation on a specified control in the vehicle is detected. Here, the specified control in the vehicle may be a control for controlling an intelligent health detection function. When the specified operation on the control for controlling the intelligent health detection function is detected, it may be determined that the data acquisition instruction is triggered. The specified operation may be an operation for enabling the intelligent health detection function, such as a click operation or a swipe operation, and no specific limitation is made in this respect.

As an approach, before it is detected whether there is a specified operation on a specified control, it may first be detected whether the driver is the vehicle owner. In the case where the driver is the vehicle owner, it is then detected whether there is a specified operation on the specified control.

In detecting whether the driver is the vehicle owner, an image of the driver may be acquired by an image acquisition device, and the acquired image of the driver may be compared with a preset image to verify whether the driver is the vehicle owner. Specifically, if the similarity between the acquired image of the driver and the preset image is greater than or equal to a preset similarity, it may be determined that the driver is the vehicle owner; conversely, if the similarity between the acquired image of the driver and the preset image is less than the preset similarity, it may be determined that the driver is not the vehicle owner. Here, the image acquisition device may be a DMS (Driver Monitoring System)/OMS (Occupancy Monitoring System) camera; the preset image may be an image of the vehicle owner, there may be multiple such images, and no specific limitation is imposed in this respect; the preset similarity is a preset minimum similarity indicating that the driver corresponding to the image is the vehicle owner.

Furthermore, when the vehicle is powered on by the driver, the DMS/OMS system may acquire an image of the driver and compare it with a preset image to verify the vehicle owner information, and transmit the verification result back to a T-Box module. The T-Box module then sends the verification result to the IVI (In-Vehicle Infotainment) end. If the verification result indicates that the driver is the vehicle owner, an "Intelligent Health Detection System" function may be further activated by clicking.

During the dynamic driving process of the vehicle, when it is detected that the intelligent health detection function is activated, it may be determined that the data acquisition instruction is triggered, and acquisition of the steering wheel angle signal of the vehicle at the current moment and acquisition of a target image at the current moment by the image acquisition device are started.

Here, the steering wheel angle signal of the vehicle at the current moment is used to represent the current rotation angle of the steering wheel. The steering wheel angle signal of the vehicle may be acquired by a steering wheel angle sensor. While the steering wheel angle signal is acquired by the steering wheel angle sensor, the target image is acquired by the image acquisition device.

Here, the target image is an image of the driver's hands holding the steering wheel at the moment the steering wheel angle signal is acquired. That is, the target image includes at least the driver's hands and the steering wheel.

In an embodiment of the present application, a plurality of ECG monitoring sensors are arranged at intervals on the steering wheel of the vehicle. The spacing between every two adjacent ECG monitoring sensors is the same. Furthermore, the plurality of ECG monitoring sensors arranged at intervals on the steering wheel can ensure that when a normal person's palm holds one side of the steering wheel, at least two ECG monitoring sensors can be covered or partially covered. Here, the ECG monitoring sensors may be conductive leather or electrode pads, and no specific limitation is made in this respect.

In step S120, based on the steering wheel angle signal and the target image, a first sensor group and a second sensor group are determined from the plurality of ECG monitoring sensors, with the first sensor group including at least two ECG monitoring sensors each having a coverage greater than a coverage threshold, and the second sensor group including at least two ECG monitoring sensors each having a coverage less than the coverage threshold.

In an embodiment of the present application, the coverage threshold may be a preset coverage value used to divide a plurality of ECG monitoring sensors each having a coverage greater than zero at the current time into two sensor groups with an equal number of sensors. The first sensor group may include at least two ECG monitoring sensors, which may be the two ECG monitoring sensors with the top largest coverages among the plurality of ECG monitoring sensors each having a coverage greater than zero at the current time. The second sensor group may also include at least two ECG monitoring sensors, which may be the remaining ECG monitoring sensors from the plurality of ECG monitoring sensors each having a coverage greater than zero at the current time after the ECG monitoring sensors in the first sensor group are excluded. The number of sensors included in the first sensor group is the same as the number of sensors included in the second sensor group.

After the steering wheel angle signal and the target image at the current moment are obtained, two sensor groups having different confidence levels may be determined from the plurality of ECG monitoring sensors based on the steering wheel angle signal and the target image at the current moment. Here, the confidence levels refer to the confidence levels of the ECG data acquired by the two sensor groups respectively. The coverage of each of the ECG monitoring sensors in the first sensor group is greater than that of each of the ECG monitoring sensors in the second sensor group. Therefore, it may be deemed that the confidence level of the ECG data acquired by the first sensor group is greater than that of the ECG data acquired by the second sensor group. That is, the confidence level of the first sensor group is greater than that of the second sensor group.

In step S130, first ECG data of the driver is acquired by the first sensor group, and second ECG data of the driver is acquired by the second sensor group.

In an embodiment of the present application, after the first sensor group and the second sensor group are determined, the ECG data of the driver may be acquired by the first sensor group and the second sensor group respectively. Here, when acquiring the first ECG data by the first sensor group, the at least two ECG monitoring sensors included in the first sensor group may be connected in parallel, and the first ECG data of the driver is acquired by the first sensor group with its sensors connected in parallel. Similarly, when acquiring the second ECG data of the driver by the second sensor group, the at least two ECG monitoring sensors included in the second sensor group may be connected in parallel, and the second ECG data of the driver may be acquired by the second sensor group with its sensors connected in parallel.

In step S140, based on the first ECG data, the second ECG data, and preset ECG data, target ECG data of the driver is determined. The preset ECG data is obtained by analyzing basic ECG data of the driver collected by an ECG acquisition module when the vehicle is in a stationary state.

In an embodiment of the present application, the preset ECG data is obtained by analyzing the basic ECG data collected by the ECG acquisition module when the vehicle is in a stationary state. At this time, the vehicle is in a stationary state, the driver holds the steering wheel with both hands, and the ECG acquisition module monitors or collects the basic ECG data of the driver in real time, and performs analysis based on stable ECG waveform data to obtain preset ECG data with high reliability. Here, the ECG acquisition module refers to a module composed of a plurality of ECG monitoring senss120ors arranged at intervals on the steering wheel. Optionally, the preset ECG data may be dynamically updated based on combined analysis of the current driver's multiple static test datasets or dynamic driving monitoring data.

As an approach, in determining the preset ECG data, while the vehicle is in a stationary state and the driver holds the steering wheel with both hands, the DMS/OMS camera may be used to identify and determine the landing position interval of the driver's left or right hand on the steering wheel. Thus, based on the landing position interval of the driver's left or right hand on the steering wheel, a first sensor group and a second sensor group may be determined from the plurality of ECG monitoring sensors. ECG data is acquired over a long period of time by the first sensor group and the second sensor group, and may be input into a pre-trained prediction model, which then may output predicted ECG data of the driver, i.e., the preset ECG data.

In an embodiment of the present application, after the first ECG data and the second ECG data at the current moment are obtained, the target ECG data of the driver may be determined based on the first ECG data, the second ECG data and the preset ECG data. The target ECG data refers to the driver's ECG data for display on a mobile terminal.

When the target ECG data of the driver is determined based on the first ECG data, the second ECG data, and the preset ECG data, a method for determining the target ECG data of the driver according to whether the first ECG data and the second ECG data are within a threshold interval is used. The threshold interval is a preset threshold interval of ECG data for a normal person.

As an approach, if each of the first ECG data and the second ECG data is within the threshold interval, the first ECG data is used as the target ECG data of the driver. If neither the first ECG data nor the second ECG data is within the threshold interval, the preset ECG data is used as the target ECG data of the driver. If either the first ECG data or the second ECG data is within the threshold interval, the ECG data within the threshold interval is used as the target ECG data of the driver.

Specifically, the first ECG data and the second ECG data are sequentially compared with two critical values of the threshold interval to determine whether the first ECG data and the second ECG data are within the threshold interval. Here, the two critical values include a first critical value and a second critical value, the first critical value being less than the second critical value. The first critical value is the minimum ECG data in the threshold interval, and the second critical value is the maximum ECG data in the threshold interval. If the first ECG data is greater than or equal to the first critical value and less than or equal to the second critical value, it may be determined that the first ECG data is within the threshold interval. Conversely, if the first ECG data is less than the first critical value or greater than the second critical value, it may be determined that the first ECG data is not within the threshold interval. Similarly, if the second ECG data is greater than or equal to the first critical value and less than or equal to the second critical value, it may be determined that the second ECG data is within the threshold interval. Conversely, if the second ECG data is less than the first critical value or greater than the second critical value, it may be determined that the second ECG data is not within the threshold interval.

Through the above method, if it is determined that both the first ECG data and the second ECG data are within the threshold interval, the first ECG data with higher confidence may be selected as the target ECG data of the driver. If it is determined that neither the first ECG data nor the second ECG data is within the threshold interval, the preset ECG data can be directly used as the target ECG data of the driver. If the first ECG data is within the threshold interval and the second ECG data is not within the threshold interval, the first ECG data may be used as the target ECG data of the driver. If the first ECG data is not within the threshold interval, and the second ECG data is within the threshold interval, the second ECG data may be used as the target ECG data of the driver. Here, when it is determined that the first ECG data and the second ECG data are not within the threshold interval, and the preset ECG data is used as the target ECG data of the driver, the duration for using the preset ECG data as the target ECG data of the driver is limited. At this time, parameters of the plurality of ECG monitoring sensors arranged at intervals on the steering wheel may be adjusted such that the ECG monitoring sensors with adjusted parameters can acquire ECG data with high reliability.

The ECG data monitoring method provided by the present application can improve the accuracy and stability of monitoring ECG data of the driver, thereby enhancing the reliability of the target ECG data of the driver.

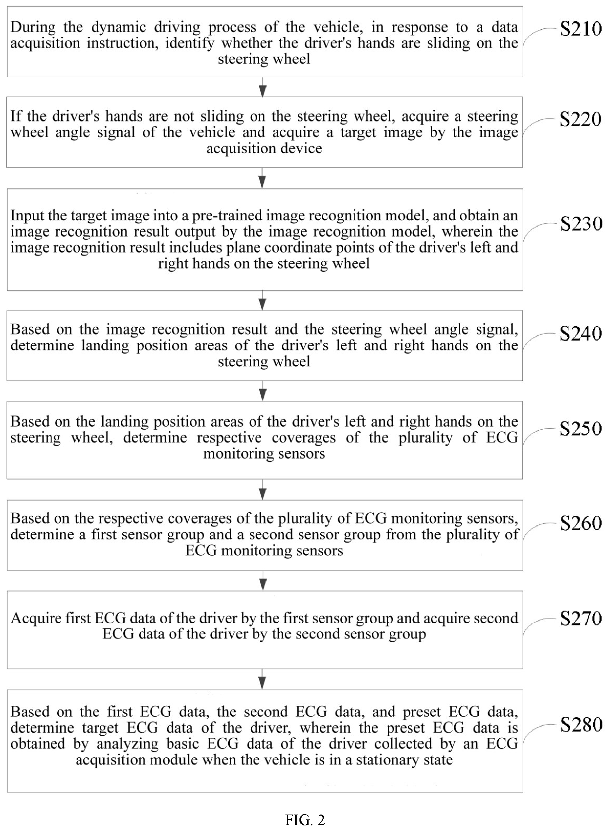

Referring to FIG. 2, an embodiment of the present application provides an ECG data monitoring method including the following steps.

In step S210, during the dynamic driving process of the vehicle, in response to a data acquisition instruction, it is identified whether the driver's hands are sliding on the steering wheel.

In an embodiment of the present application, when it is detected that the vehicle is in a dynamic driving process, if a data acquisition instruction is detected, it is necessary to first identify, by a DMS/OMS camera, whether the driver's hands are sliding on the steering wheel. Specifically, multiple images may be continuously acquired by the DMS/OMS camera, and based on the continuously acquired multiple images, it is identified whether the landing positions of the driver's hands on the steering wheel have changed, thereby determining whether the driver's hands are sliding on the steering wheel.

In step S220, if the driver's hands are not sliding on the steering wheel, a steering wheel angle signal of the vehicle is acquired, and a target image is acquired by an image acquisition device.

In an embodiment of the present application, in determining whether the driver's hands are sliding on the steering wheel through the above method, it may be identified, based on multiple continuously acquired images, whether the landing positions of the driver's hands on the steering wheel have changed. If there is a change, it may be determined that the driver's hands are sliding on the steering wheel; conversely, it may be determined that the driver's hands are not sliding on the steering wheel.

When it is determined that the driver's hands are not slipping on the steering wheel, the steering wheel angle signal of the vehicle at the current moment may be acquired, and a target image at the current moment may be acquired by the image acquisition device.

In step S230, the target image is input into a pre-trained image recognition model and an image recognition result output by the image recognition model is obtained. The image recognition result includes plane coordinate points of the driver's left and right hands on the steering wheel.

In an embodiment of the present application, the image recognition model is a pre-trained model for recognizing plane coordinate points of the driver's hands on the steering wheel. After the target image at the current moment is obtained, the target image may be input into the pre-trained image recognition model to obtain an image recognition result that is output by the image recognition model and corresponds to the target image.

Before the target image is input into the pre-trained image recognition model, a training dataset is acquired. The training dataset includes a plurality of training images, each of which is annotated with plane coordinate points of the driver's hands on the steering wheel. The training dataset is input into a model to be trained, and the model to be trained is iteratively trained until a training termination condition is met, thereby obtaining the image recognition model. Here, the training termination condition may be set as the number of training iterations reaching a preset number, or the training termination condition may be set as a loss value reaching a preset loss value and no longer decreasing.

In an embodiment of the present application, the plane coordinate points of the driver's left and right hands on the steering wheel may be used to represent the positions of the driver's left and right hands on the steering wheel.

In step S240, based on the image recognition result and the steering wheel angle signal, the landing position areas of the driver's left and right hands on the steering wheel are determined.

In an embodiment of the present application, after the image recognition result of the target image is obtained by the image recognition model, the image recognition result may be combined with the steering wheel angle signal at the current moment to determine the landing position areas of the driver's left and right hands on the steering wheel at the current moment relative to a zero angle. The landing position areas of the driver's left and right hands on the steering wheel may be determined by boundary coordinate points of the driver's left and right hands.

In step S250, based on the landing position areas of the driver's left and right hands on the steering wheel, the respective coverages of the plurality of ECG monitoring sensors are determined.

In an embodiment of the present application, after the landing position areas of the driver's left and right hands on the steering wheel are determined, the ECG monitoring sensors covered by the left hand may be determined according to the landing position area of the driver's left hand, and the ECG monitoring sensors covered by the right hand may be determined according to the landing position area of the driver's right hand. This is possible because the positions of the ECG sensors disposed on the steering wheel are known to the vehicle and the sizes of the ECG monitoring sensors are also fixed. Therefore, after the ECG monitoring sensors covered by the driver's left hand and the ECG monitoring sensors covered by the driver's right hand are determined, respective coverages corresponding to the ECG monitoring sensors covered by the left hand may be further determined according to the landing position area of the driver's left hand, and the respective coverages corresponding to the ECG monitoring sensors covered by the right hand may be determined according to the landing position area of the driver's right hand. The respective coverages corresponding to the remaining uncovered ECG monitoring sensors on the steering wheel are zero. Here, each of the coverages may be understood as the contact area between the driver's left or right hand and the corresponding ECG monitoring sensor. The greater the coverage, the larger the contact area between the driver's left or right hand and the ECG monitoring sensor; conversely, the smaller the coverage, the smaller the contact area between the driver's left or right hand and the ECG monitoring sensor.

It can be understood that the larger the contact area of an ECG monitoring sensor, the more stable the acquired ECG data and the higher the tolerance rate.

In step S260, based on the respective coverages of the plurality of ECG monitoring sensors, a first sensor group and a second sensor group are determined from the plurality of ECG monitoring sensors.

In an embodiment of the present application, after the respective coverages of the plurality of ECG monitoring sensors are obtained through the above method, the first sensor group and the second sensor group may be determined from the plurality of ECG monitoring sensors based on the respective coverages of the ECG monitoring sensors.

As an approach, based on the respective coverages of the plurality of ECG monitoring sensors, a plurality of target ECG monitoring sensors are determined from the plurality of ECG monitoring sensors. The plurality of target ECG monitoring sensors are ECG monitoring sensors each having a coverage greater than zero among the plurality of ECG monitoring sensors. Among the plurality of target ECG monitoring sensors, target ECG monitoring sensors each having a coverage greater than a coverage threshold are classified into a first sensor group. Among the plurality of target ECG monitoring sensors, target ECG monitoring sensors each having a coverage less than the coverage threshold are classified into a second sensor group.

Specifically, the ECG monitoring sensors each having a coverage greater than zero and greater than the coverage threshold among the plurality of ECG monitoring sensors are classified into the first sensor group; the ECG monitoring sensors each having a coverage greater than zero and less than the coverage threshold among the plurality of ECG monitoring sensors are classified into the second sensor group.

As another approach, after the respective coverages of the plurality of ECG monitoring sensors are obtained, the plurality of ECG monitoring sensors may be sorted in a descending or ascending order of coverage to obtain a sorted plurality of ECG monitoring sensors. Among the sorted plurality of ECG monitoring sensors, the ECG monitoring sensors ranked at a first specified position are classified into the first sensor group. Among the sorted plurality of ECG monitoring sensors, the ECG monitoring sensors ranked at a second specified position are classified into the second sensor group. Here, if sorting is performed in a descending order of coverage, the first specified position is before the second specified position. For example, the first specified position may be the top two positions, and the second specified position may be the third and fourth positions. Conversely, the first specified position is after the second specified position.

In step S270, first ECG data of the driver is acquired by the first sensor group, and second ECG data of the driver is acquired by the second sensor group.

In step S280, based on the first ECG data, the second ECG data, and preset ECG data, target ECG data of the driver is determined. The preset ECG data is obtained by analyzing basic ECG data of the driver collected by an ECG acquisition module when the vehicle is a stationary state.

In an embodiment of the present application, after the target ECG data is obtained, a more comprehensive vehicle owner health profile may be generated based on the target ECG data. Optionally, the target ECG data of the driver may also be uploaded to a cloud, such that the driver can view the target ECG data in real time on a mobile terminal.

The ECG data monitoring method provided by the present application can improve the accuracy and stability of monitoring ECG data of the driver, thereby enhancing the reliability of the target ECG data of the driver.

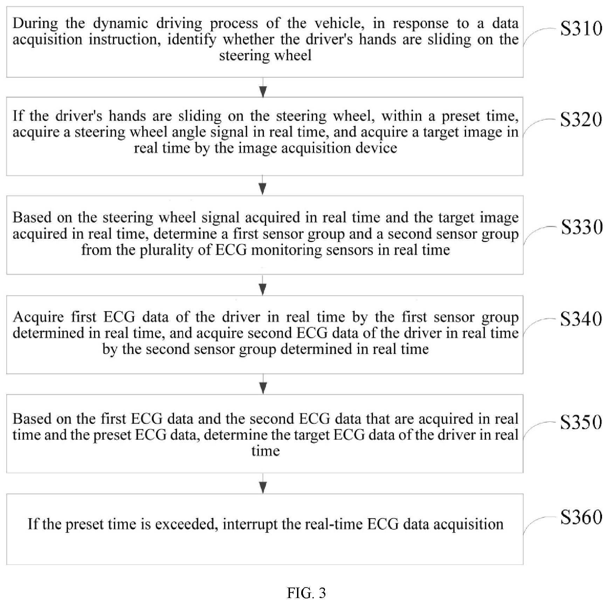

Referring to FIG. 3, an embodiment of the present application provides an ECG data monitoring method including the following steps.

In step S310, during the dynamic driving process of the vehicle, in response to a data acquisition instruction, it is identified whether the driver's hands are sliding on the steering wheel.

In step S320, if the driver's hands are sliding on the steering wheel, within a preset time, a steering wheel angle signal is acquired in real time and a target image is acquired in real time by the image acquisition device.

In an embodiment of the present application, it may be identified, based on multiple continuously acquired images, whether the landing positions of the driver's hands on the steering wheel have changed. If there is a change, it may be determined that the driver's hands are sliding on the steering wheel; conversely, it may be determined that the driver's hands are not sliding on the steering wheel.

Through the above method, if it is determined that the driver's hands are sliding on the steering wheel (a steering wheel turning scenario), the sensors with which the driver's left or right hand is in contact will be constantly switching. In this case, the steering wheel angle signal and the target image may be acquired in real time within a preset time period.

In step S330, based on the steering wheel signal acquired in real time and the target image acquired in real time, a first sensor group and a second sensor group are determined from the plurality of ECG monitoring sensors in real time.

In an embodiment of the present application, the first sensor group and the second sensor group may be determined in real time based on the steering wheel angle signal and the target image that are acquired in real time.

In step S340, first ECG data of the driver is acquired in real time by the first sensor group determined in real time, and second ECG data of the driver is acquired in real time by the second sensor group determined in real time.

In step S350, based on the first ECG data and the second ECG data that are acquired in real time, and preset ECG data, target ECG data of the driver is determined in real time.

In an embodiment of the present application, ECG data may be acquired in real time by the first sensor group and the second sensor group that are determined in real time. The ECG data acquired in real time is used to automatically generate a temporary segment of target ECG data with high reliability for display based on a fusion process of the first ECG data, the second ECG data and the preset ECG data, thereby ensuring uninterrupted ECG data acquisition. The fusion process of the first ECG data, the second ECG data, and the preset ECG data refers to the fusion process described in step S140.

In step S360, if the preset time is exceeded, the real-time ECG data acquisition is interrupted.

In an embodiment of the present application, when acquisition of ECG data by the first sensor group and the second sensor group is started, a timer is started. When the timer exceeds the preset time, the real-time ECG data acquisition is interrupted.

The ECG data monitoring method provided by the present application can improve the accuracy and stability of monitoring ECG data of the driver, thereby enhancing the reliability of the target ECG data of the driver.

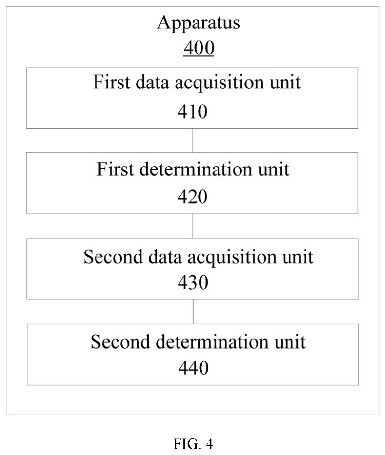

Referring to FIG. 4, an embodiment of the present application provides an ECG data monitoring apparatus 400 including the following components.

A first data acquisition unit 410 is configured to: during a dynamic driving process of a vehicle, in response to a data acquisition instruction, acquire a steering wheel angle signal of the vehicle and acquire a target image by an image acquisition device. The target image is an image of the driver's hands holding the steering wheel. A plurality of ECG monitoring sensors are arranged at intervals on the steering wheel.

As an approach, the first data acquisition unit 410 is specifically configured to: during the dynamic driving process of the vehicle, in response to a data acquisition instruction, identify whether the driver's hands are sliding on the steering wheel; if the driver's hands are not sliding on the steering wheel, acquire a steering wheel angle signal of the vehicle and acquire a target image by the image acquisition device.

As another approach, the first data acquisition unit 410 is specifically configured to, if the driver's hands are sliding on the steering wheel, within a preset time, acquire a steering wheel angle signal in real time and acquire a target image in real time by the image acquisition device.

A first determination unit 420 is configured to: based on the steering wheel angle signal and the target image, determine a first sensor group and a second sensor group from the plurality of ECG monitoring sensors. The first sensor group includes at least two ECG monitoring sensors each having a coverage greater than a coverage threshold, and the second sensor group includes at least two ECG monitoring sensors each having a coverage less than the coverage threshold.

As an approach, the first determination unit 420 is specifically configured to: input the target image into a pre-trained image recognition model, and obtain an image recognition result output by the image recognition model, the image recognition result including plane coordinate points of the driver's left and right hands on the steering wheel; based on the image recognition result and the steering wheel angle signal, determine landing position areas of the driver's left and right hands on the steering wheel; based on the landing position areas of the driver's left and right hands on the steering wheel, determine respective coverages of the plurality of ECG monitoring sensors; and based on the respective coverages of the plurality of ECG monitoring sensors, determine a first sensor group and a second sensor group from the plurality of ECG monitoring sensors.

Furthermore, the first determination unit 420 is specifically configured to: based on the respective coverages of the plurality of ECG monitoring sensors, determine a plurality of target ECG monitoring sensors from the plurality of ECG monitoring sensors, the plurality of target ECG monitoring sensors being ECG monitoring sensors each having a coverage greater than zero among the plurality of ECG monitoring sensors; among the plurality of target ECG monitoring sensors, classify the target ECG monitoring sensors each having a coverage greater than a coverage threshold into the first sensor group; and among the plurality of target ECG monitoring sensors, classify the target ECG monitoring sensors each having a coverage less than the coverage threshold into the second sensor group.

As another approach, the first determination unit 420 is specifically configured to: based on the steering wheel signal acquired in real time and the target image acquired in real time, determine the first sensor group and the second sensor group from the plurality of ECG monitoring sensors in real time.

A second data acquisition unit 430 is configured to acquire first ECG data of the driver by the first sensor group and acquire second ECG data of the driver by the second sensor group.

As an approach, the second data acquisition unit 430 is specifically configured to: acquire the first ECG data of the driver in real time by the first sensor group determined in real time, and acquire the second ECG data of the driver in real time by the second sensor group determined in real time.

A second determination unit 440 is configured to determine target ECG data of the driver based on the first ECG data, the second ECG data, and preset ECG data. The preset ECG data is obtained by analyzing basic ECG data of the driver collected by an ECG acquisition module when the vehicle is in a stationary state.

As an approach, the second determination unit 440 is specifically configured to: if each of the first ECG data and the second ECG data is within a threshold interval, use the first ECG data as the target ECG data of the driver; if neither the first ECG data nor the second ECG data is within the threshold interval, use the preset ECG data as the target ECG data of the driver; and if either the first ECG data or the second ECG data is within the threshold interval, use the ECG data within the threshold interval as the target ECG data of the driver.

As another approach, the second determination unit 440 is specifically configured to determine the target ECG data of the driver in real time, based on the first ECG data and the second ECG data that are acquired in real time and the preset ECG data.

Optionally, the second determination unit 440 is specifically configured to interrupt the real-time ECG data acquisition if the preset time is exceeded.

It should be noted that the apparatus embodiments in the present application correspond to the method embodiments described above. The specific principles in the apparatus embodiments may be found in the content of the method embodiments described above, and will not be repeated here.

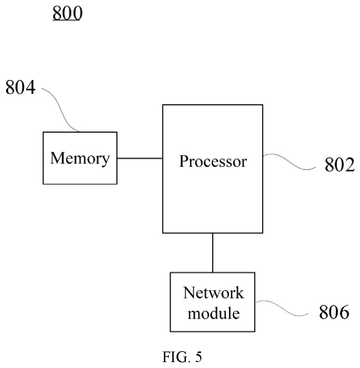

A vehicle provided by the present application will be described below with reference to FIG. 5.

Referring to FIG. 5, based on the above-described ECG data monitoring method and apparatus, an embodiment of the present application further provides a vehicle 800 capable of executing the aforementioned ECG data monitoring method. The vehicle 800 includes one or more processors 802 (only one is shown in the figure), a memory 804 and a network module 806 that are coupled to each other. The memory 804 stores a program capable of executing the content in the foregoing embodiments, and the processor 802 can execute the program stored in a memory 804.

Here, each processor 802 may include one or more processing cores. The processor 802 uses various interfaces and lines to connect various parts within the vehicle 800, and executes various functions of the vehicle 800 and processes data by running or executing instructions, programs, code sets or instruction sets stored in the memory 804 and by calling data stored in the memory 804. Optionally, the processor 802 may be implemented in at least one of the following hardware forms: digital signal processor (DSP), field-programmable gate array (FPGA), and programmable logic array (PLA). The processor 802 may integrate one, or any combination, of a central processing unit (CPU), a graphics processing unit (GPU), a modem and the like. Here, the CPU mainly handles an operating system, a user interface, application programs, and the like; the GPU is responsible for rendering and drawing display content; and the modem is used for processing wireless communications. It can be understood that the aforementioned modem may not be integrated into the processor 802, and may be implemented separately as a single communication chip.

The memory 804 may include a random access memory (RAM) or a read-only memory. The memory 804 can be used to store instructions, programs, codes, code sets, or instruction sets. The memory 804 may include a program storage area and a data storage area. The program storage area may store instructions for implementing an operating system, instructions for implementing at least one function (such as touch control function, a sound playback function, an image playback function, and the like), instructions for implementing the various method embodiments described below, and other instructions. The storage data area may also store data (such as a phone book, audio and video data, chat history data, and the like) created during the use of the vehicle 800.

The network module 806 is configured to receive and transmit electromagnetic waves, and realize mutual conversion between electromagnetic waves and electrical signals, thereby communicating with a communication network or other devices, for example, communicating with vehicles. The network module 806 may include various existing circuit elements for performing these functions, such as an antenna, a radio frequency transceiver, a digital signal processor, an encryption/decryption chip, a subscriber identity module (SIM) card, a memory, and the like. The network module 806 may communicate with various networks such as the Internet, an intranet, and a wireless network, or communicate with other devices via a wireless network. The aforementioned wireless network may include a cellular telephone network, a wireless local area network (WLAN), or a metropolitan area network (MAN). For example, the network module 806 can exchange information with a base station.

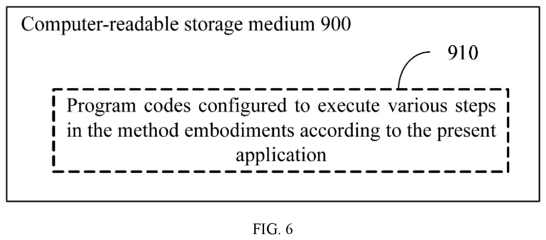

Referring to FIG. 6, a structural block diagram of a computer-readable storage medium provided in an embodiment of the present application is showed. Program codes are stored in the computer-readable storage medium 900, and can be called by a processor to execute the methods described in the above method embodiments.

The computer-readable storage medium 900 may be an electronic memory such as a flash memory, an EEPROM (electrically erasable programmable read-only memory), an EPROM, a hard disk, or a ROM. Optionally, the computer-readable storage medium 900 includes a non-transitory computer-readable storage medium. The computer-readable storage medium 900 has storage space for program codes 910 that are used for executing any of the method steps in the above methods. These program codes can be read from or written into one or more computer program products. The program codes 910 can be compressed, for example, in an appropriate form.

The present application provides an ECG data monitoring method, an apparatus, a vehicle, and a storage medium. During a dynamic driving process of a vehicle, in response to a data acquisition instruction, a steering wheel angle signal is acquired, and a target image is acquired by an image acquisition device. The target image is an image of the driver's hands holding the steering wheel. A plurality of ECG monitoring sensors are arranged at intervals on the steering wheel. Then, based on the steering wheel angle signal and the target image, a first sensor group and a second sensor group are determined from the plurality of ECG monitoring sensors, with the first sensor group including at least two ECG monitoring sensors each having a coverage greater than a coverage threshold, and the second sensor group including at least two ECG monitoring sensors each having a coverage less than the coverage threshold. Next, first ECG data of the driver is acquired by the first sensor group and second ECG data of the driver is acquired by the second sensor group. Finally, target ECG data of the driver is determined based on the first ECG data, the second ECG data, and preset ECG data. The preset ECG data is obtained by analyzing basic ECG data of the driver collected by an ECG acquisition module when the vehicle is in a stationary state. Through the above method, the accuracy and stability of monitoring ECG data of the driver can be improved, thereby enhancing the reliability of the target ECG data of the driver.

The embodiments of the present application have been described above with reference to the accompanying drawings. However, the present application is not limited to the specific implementations described above. The specific implementations described above are merely illustrative and not restrictive. Inspired by the present application, those of ordinary skill in the art can make many other forms without departing from the gist of the present application and the scope claimed by the claims, all of which fall within the protection scope of the present application.

## Claims

1. A method for monitoring electrocardiogram (ECG) data, **characterized by** comprising:
during a dynamic driving process of a vehicle, in response to a data acquisition instruction, acquiring a steering wheel angle signal of the vehicle and acquiring a target image by an image acquisition device, wherein the target image is an image of a driver's hands holding the steering wheel, and a plurality of ECG monitoring sensors are arranged at intervals on the steering wheel;
based on the steering wheel angle signal and the target image, determining a first sensor group and a second sensor group from the plurality of ECG monitoring sensors, wherein the first sensor group comprises at least two ECG monitoring sensors each having a coverage greater than a coverage threshold, and the second sensor group comprises at least two ECG monitoring sensors each having a coverage less than the coverage threshold;
acquiring first ECG data of the driver by the first sensor group, and acquiring second ECG data of the driver by the second sensor group; and
based on the first ECG data, the second ECG data, and preset ECG data, determining target ECG data of the driver, wherein the preset ECG data is obtained by analyzing basic ECG data of the driver collected by an ECG acquisition module when the vehicle is in a stationary state.

2. The method according to claim 1, wherein based on the steering wheel angle signal and the target image, determining a first sensor group and a second sensor group from the plurality of ECG monitoring sensors comprises:
inputting the target image into a pre-trained image recognition model, and obtaining an image recognition result output by the image recognition model, wherein the image recognition result comprises plane coordinate points of the driver's left and right hands on the steering wheel;
based on the image recognition result and the steering wheel angle signal, determining landing position areas of the driver's left and right hands on the steering wheel;
based on the landing position areas of the driver's left and right hands on the steering wheel, determining respective coverages of the plurality of ECG monitoring sensors; and
based on the respective coverages of the plurality of ECG monitoring sensors, determining a first sensor group and a second sensor group from the plurality of ECG monitoring sensors.

3. The method according to claim 2, wherein based on the respective coverages of the plurality of ECG monitoring sensors, determining a first sensor group and a second sensor group from the plurality of ECG monitoring sensors comprises:
based on the respective coverages of the plurality of ECG monitoring sensors, determining a plurality of target ECG monitoring sensors from the plurality of ECG monitoring sensors, wherein the plurality of target ECG monitoring sensors are ECG monitoring sensors each having a coverage greater than zero among the plurality of ECG monitoring sensors;
among the plurality of target ECG monitoring sensors, classifying the target ECG monitoring sensors each having a coverage greater than a coverage threshold into the first sensor group; and
among the plurality of target ECG monitoring sensors, classifying the target ECG monitoring sensors each having a coverage less than the coverage threshold into the second sensor group.

4. The method according to claim 1, wherein based on the first ECG data, the second ECG data, and the preset ECG data, determining target ECG data of the driver comprises:
if each of the first ECG data and the second ECG data is within a threshold interval, using the first ECG data as the target ECG data of the driver;
if neither the first ECG data nor the second ECG data is within the threshold interval, using the preset ECG data as the target ECG data of the driver; and
if either the first ECG data or the second ECG data is within the threshold interval, using the ECG data within the threshold interval as the target ECG data of the driver.

5. The method according to claim 1, wherein during the dynamic driving process of the vehicle, in response to a data acquisition instruction, acquiring a steering wheel angle signal of the vehicle and acquiring a target image by the image acquisition device comprises:
during the dynamic driving process of the vehicle, in response to a data acquisition instruction, identifying whether the driver's hands are sliding on the steering wheel; and
if the driver's hands are not sliding on the steering wheel, acquiring a steering wheel angle signal of the vehicle and acquiring a target image by the image acquisition device.

6. The method according to claim 5, wherein the method further comprises:
if the driver's hands are sliding on the steering wheel, within a preset time, acquiring a steering wheel angle signal in real time, and acquiring a target image in real time by the image acquisition device;
based on the steering wheel angle signal and the target image, determining a first sensor group and a second sensor group from the plurality of ECG monitoring sensors comprises:
based on the steering wheel signal acquired in real time and the target image acquired in real time, determining a first sensor group and a second sensor group from the plurality of ECG monitoring sensors in real time;
the acquiring first ECG data of the driver by the first sensor group and acquiring second ECG data of the driver by the second sensor group comprises:
acquiring first ECG data of the driver in real time by the first sensor group determined in real time, and acquiring second ECG data of the driver in real time by the second sensor group determined in real time;
based on the first ECG data, the second ECG data, and the preset ECG data, the determining the target ECG data of the driver comprises:
based on the first ECG data and the second ECG data that are acquired in real time, and the preset ECG data, determining the target ECG data of the driver in real time.

7. The method according to claim 6, wherein the method further comprises:
if the preset time is exceeded, interrupting the real-time ECG data acquisition.

8. An apparatus for monitoring electrocardiogram (ECG) data, **characterized by** comprising:
a first data acquisition unit configured to: during a dynamic driving process of a vehicle, in response to a data acquisition instruction, acquire a steering wheel angle signal of the vehicle and acquire a target image by an image acquisition device, wherein the target image being an image of a driver's hands holding the steering wheel, and a plurality of ECG monitoring sensors are arranged at intervals on the steering wheel;
a first determination unit configured to, based on the steering wheel angle signal and the target image, determine a first sensor group and a second sensor group from the plurality of ECG monitoring sensors, wherein the first sensor group comprises at least two ECG monitoring sensors each having a coverage greater than a coverage threshold, and the second sensor group comprises at least two ECG monitoring sensors each having a coverage less than the coverage threshold;
a second data acquisition unit configured to acquire first ECG data of the driver by the first sensor group and acquire second ECG data of the driver by the second sensor group; and
a second determination unit configured to determine target ECG data of the driver based on the first ECG data, the second ECG data, and preset ECG data, wherein the preset ECG data is obtained by analyzing basic ECG data of the driver collected by an ECG acquisition module when the vehicle is in a stationary state.

9. A vehicle, **characterized by** comprising one or more processors, wherein one or more programs are stored in a memory and configured to be executed by the one or more processors to perform the method according to any one of claims 1 to 7.

10. A computer-readable storage medium, **characterized by** storing program codes, wherein when executed by a processor, the program codes perform the method according to any one of claims 1 to 7.
